(19) Europäisches Patentamt
European Patent Office
Office européen des brevets

(11) **EP 3 981 366 B1**

(12) **EUROPEAN PATENT SPECIFICATION**

(45) Date of publication and mention of the grant of the patent:
**01.05.2024 Bulletin 2024/18**

(21) Application number: **21164406.7**

(22) Date of filing: **23.03.2021**

(51) International Patent Classification (IPC):
***A61F 2/66*** *(2006.01)*

(52) Cooperative Patent Classification (CPC):
**A61F 2/66;** A61F 2002/6664

(54) **PROSTHETIC FOOT**

FUSSPROTHESE

PIED PROTHÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2020 CN 202011071231**

(43) Date of publication of application:
**13.04.2022 Bulletin 2022/15**

(73) Proprietor: **Jilin University
Changchun, Jilin 130012 (CN)**

(72) Inventors:
• **REN, Lei**
  **Changchun City, Jilin Province (CN)**
• **WANG, Kunyang**
  **Changchun City, Jilin Province (CN)**
• **XUE, Yifeng**
  **Changchun City, Jilin Province (CN)**
• **QIAN, Zhihui**
  **Changchun City, Jilin Province (CN)**
• **XIU, Haohua**
  **Changchun City, Jilin Province (CN)**
• **LIANG, Wei**
  **Changchun City, Jilin Province (CN)**
• **REN, Luquan**
  **Changchun City, Jilin Province (CN)**

(74) Representative: **Inchingalo, Simona
Bugnion S.p.A.
Viale Lancetti, 17
20158 Milano (IT)**

(56) References cited:
**WO-A1-2016/022699    WO-A2-2007/035894
US-A1- 2015 289 996**

**Description**

**Technical Field**

The present invention relates to a prosthetic foot as set out in the claims.

**Background**

With a rapid increase of modern social problems of disease, aging, traffic accidents, etc., a number of amputees have been increasing. Installation of a prosthesis is an effective engineering method to make up for a function loss caused by a missing limb, so worldwide attention has been paid to a research and development of prosthetic equipment and life support equipment for disabled people.

In recent years, carbon fiber-reinforced composite materials have been widely used in a field of prostheses because of their advantages of being lighter and more attractive. At present, a carbon fiber prosthetic foot is basically straight in a transverse direction and made by taking longitudinal arch features of a human foot as a reference, carbon fibers are mainly distributed in a length direction of a prosthetic foot, which ensures a strength of the prosthetic foot in the length direction, but makes a strength of the prosthetic foot weak in a transverse direction, and a market generally use thickened carbon fiber plates to solve a strength problem, which increases a weight and cost of the prosthetic foot and reduces a use effect and a comfort level of a prosthesis.

Researches show that a transverse arch of a human foot plays an important role in ensuring the overall stiffness of the human foot, which is a key factor in ensuring normal walking of a human body. Therefore, it is necessary to provide a novel prosthetic foot combining the longitudinal arch and transverse arch features of a human foot; on the one hand, the novel prosthetic foot can play a good role in weight bearing and supporting and propelling, so as to ensure rational strength and rational rigidity distribution of a bearing part of the prosthetic foot; and on the other hand, the prosthetic foot is light as much as possible, so as to improve a using effect and comfort level of a prosthesis.

The document US 2015/0289996 A1 discloses an adjustable length prosthetic foot, a foot prosthesis that includes at least one spring element, an attachment member, and a heel member. The at least one spring element has a toe end portion, a heel end portion, an upper surface, and a lower surface. The attachment member is mounted to the upper surface and is configured to connect the foot prosthesis to a lower limb prosthesis component. A position of the attachment member is adjustable along a length of the at least one spring element. The heel member is mounted below the lower surface of the at least one spring element, and a position of the heel member is adjustable along the length of the at least one spring element.

**Summary**

As for defects of an existing prosthetic foot, the present invention provides a prosthetic foot as set out in the claims. Three-dimensional curvature features of a longitudinal arch and a transverse arch of a human foot are combined into a design of a prosthetic carbon fiber foot, which changes a conventional design of the carbon fiber foot straight in a transverse direction, on one hand, weight of the prosthetic foot is reduced while overall rigidity of the prosthetic foot is improved, and on the other hand, the rigidity distribution of the foot is changed in a gradient manner due to different curvatures of different transverse positions of the prosthetic foot, which dynamically meets different requirements of different road conditions and different walking stages on the rigidity of different parts of the prosthetic foot.

Some embodiments of the present invention include a forefoot carbon fiber curved plate A, a heel carbon fiber curved plate B, a connection assembly C, a first bolt set 1, a second bolt set 2 and a third bolt set 3, wherein a square cone joint 6 of the connection assembly C, an upper connection base 7 of the connection assembly C, a rear part of the forefoot carbon fiber curved plate A, an upper part of the heel carbon fiber curved plate B and a lower abutting plate 8 of the connection assembly C are sequentially arranged from top to bottom; a square table 6b of the square cone joint 6 in the connection assembly C is arranged in a square shallow recessed groove 7d of the upper connection base 7 and is fixedly connected by the first bolt set 1, a center of the square cone joint 6 is provided with a stepped hole 6a, the first bolt set 1 is penetrated in the stepped hole 6a; a third left hole 7c of a first arc side wing 7b of the upper connection base 7 in the connection assembly C, a first left hole 4a of the forefoot carbon fiber curved plate A, a second left hole 5a of the heel carbon fiber curved plate B and a fourth left hole 8a of the lower abutting plate 8 in the connection assembly C are fixedly connected by the third bolt set 3; a third right hole 7g of a second arc side wing 7f of the upper connection base 7 in the connection assembly C, a first right hole 4b of the forefoot carbon fiber curved plate A, a second right hole 5b of the heel carbon fiber curved plate B and a fourth right hole 8b of the lower abutting plate 8 in the connection assembly C are fixedly connected by the second bolt set 2; and a forefoot longitudinal curve G of the forefoot carbon fiber curved plate A and a heel longitudinal curve H of the heel carbon fiber curved plate B integrally form a longitudinal arch shape.

The forefoot carbon fiber curved plate A includes a rear arc portion 4c, a middle curved surface portion 4d, a left foretoe 4e, a right foretoe 4f, and the forefoot longitudinal curve G, wherein the first left hole 4a and the first right hole 4b are provided in a rear part of the rear arc portion 4c; a shape of a transverse curved surface of the forefoot carbon fiber curved plate A is determined by a first cross-sectional curve D in a rear of the forefoot carbon fiber curved plate A, a second cross-sectional curve E in a middle of the forefoot carbon fiber curved plate A and a third cross-sectional curve F in a front of the curved plate, wherein

the first cross-sectional curve D is an arc curve, and has an arc radius $r_1$ of 30-35 mm and a central angle $\theta_1$ of 50°-60°;

a mathematical expression of the second cross-sectional curve E is:

$$y = -0.00009x^3 + 0.015x^2 + 0.02x + 31.5 \; ;$$

and a mathematical expression of the third cross-sectional curve F is:

$$y = -0.000055x^3 + 0.006x^2 + 0.05x + 14.5 \; .$$

Shapes of the second cross-sectional curve E and the third cross-sectional curve F are drawn according to a basic shape of the transverse arch of the human body, and have a larger curvature of inner sides and a smaller curvature of outer sides; the rear arc portion 4c is an arc cylindrical surface which is controlled by the first cross-sectional curve D and has a thickness of 4-5 mm, and the first left hole 4a and the first right hole 4b are provided in a rear part of the rear arc portion 4c; the middle curved surface portion 4d is a curved surface formed by the first cross-sectional curve D and the second cross-sectional curve E; an entire front part of the forefoot carbon fiber curved plate A is a curved surface formed by the second cross-sectional curve E and the third cross-sectional curve F, and a bottommost part thereof is an upward straight arc surface and is divided into the left foretoe 4e and the right foretoe 4f by a thin groove 41 in a middle of a front part of the forefoot carbon fiber curved plate A; a thickness from the middle curved surface portion 4d to top ends of the left foretoe 4e and the right foretoe 4f is uniformly changed, from large to small, from back to front, a maximum thickness is identical to a thickness of the rear arc portion 4c, and a minimum thickness is 2.5 mm; and the forefoot longitudinal curve G includes a first forefoot longitudinal curve section 4g and a second forefoot longitudinal curve section 4h, the first forefoot longitudinal curve section 4g is a straight line section with an inclination angle of 23°-30°, and the second forefoot longitudinal curve section 4h is an arc, having an arc radius $r_2$ of 70-80 mm and a central angle $\theta_2$ of 50°-60°, and being located at a tail end of the front part of the forefoot longitudinal curve G, and the first forefoot longitudinal curve section 4g and the second forefoot longitudinal curve section 4h are in smooth tangent connection.

The heel carbon fiber curved plate B includes an upper arc portion 5c, a middle transition portion 5d, a lower curved plate portion 5e and a heel longitudinal curve H, wherein the upper arc portion 5a is an arc plate with transversely distributed curvature and has a shape matched with a first cross-sectional curve D in the forefoot carbon fiber curved plate A; two symmetrical through holes, that is, the second left hole 5a and the second right hole 5b, are provided in a left side and a right side of the upper arc portion 5c; a bottommost part of the lower curved plate portion 5e is an upward transverse straight arc surface; the upper arc portion 5c and the lower curved plate potion 5e are smoothly connected together by the middle transition portion 5d; a thickness of a curved plate of the upper arc portion 5c of the heel carbon fiber curved plate B is uniform and is 5-7 mm; and a thickness of the middle transition portion 5d and a thickness of the lower curved plate part 5e are uniformly changed, from large to small, from back to front, a maximum thickness of each of the middle transition portion 5d and the lower curved plate part 5e is consistent with a thickness of the upper arc portion 5c, and a minimum thickness is 3 mm.

The heel longitudinal curve H includes a first heel longitudinal curve section 5f and a second heel longitudinal curve section 5g, wherein the first heel longitudinal curve section 5f includes an inclined straight line and an arc, wherein the arc has a radius $r_3$ of 70-80 mm and a central angle $\theta_3$ of 130°-150°, and the first heel longitudinal curve section is located on an upper middle part of the heel longitudinal curve H, wherein the second heel longitudinal curve section 5g includes an inclined straight line and an arc, having an arc radius $r_4$ of 60-70 mm and a central angle $\theta_4$ of 8°-12°, and is located on a lower part of the heel longitudinal curve H, and the first heel longitudinal curve section 5f and the second heel longitudinal curve section 5g are in smooth tangent connection by the straight line.

The connection assembly C includes the square cone joint 6, the upper connection base 7 and the lower abutting plate 8, wherein a lower part of the square cone joint 6 is the square table 6b; the upper connection base 7 includes a middle platform 7a, and the first arc side wing 7b and the second arc side wing 7f which are symmetrically arranged left and right, wherein the middle platform 7a is a hollow triangular pyramid frustum horizontally disposed in a left-right direction, the square shallow recessed groove 7d and a middle hole 7e are provided in a top face of the middle platform 7a, the third left hole 7c is provided in the first arc side wing 7b, and the third right hole 7g is provided in the second arc side wing 7f; the fourth left hole 8a and the fourth right hole 8b are provided in the lower abutting plate 8; and the square cone joint 6, the upper connection base 7 and the lower abutting plate 8 are sequentially arranged from top to bottom.

Some embodiments of the present invention provide a prosthetic foot, including a forefoot carbon fiber curved plate, a heel carbon fiber curved plate, a connection assembly C, a first fastening assembly, a second fastening assembly, and a third fastening assembly; a square cone joint of the connection assembly is disposed in a square shallow recessed groove of an upper connection base and is connected by the first fastening assembly, a center of the square cone joint is provided with a stepped hole, the first fastening assembly is penetrated in the stepped hole; a first arc side wing of the upper connection base in the connection assembly, the forefoot carbon fiber curved plate, the heel carbon fiber curved plate and a lower abutting plate in the connection assembly are fixedly connected by the third fastening assembly; a second arc side wing of the upper connection base in the connection assembly, the forefoot carbon fiber curved plate, the heel carbon fiber curved plate and the lower abutting plate in the connection assembly are fixedly connected by the second fastening assembly; and the forefoot carbon fiber curved plate and the heel carbon fiber curved plate form a longitudinal arch shape, and the forefoot carbon fiber curved plate forms a transverse arch shape.

In some embodiments, the first fastening assembly is a first bolt set, the second fastening assembly is a second bolt set, and the third fastening assembly is a third bolt set.

In some embodiments, the square cone joint of the connection assembly, the upper connection base of the connection assembly, the rear part of the forefoot carbon fiber curved plate, the upper part of the heel carbon fiber curved plate and the lower abutting plate of the connection assembly are sequentially arranged from top to bottom.

In some embodiments, the forefoot carbon fiber curved plate has a forefoot longitudinal curve, and the heel carbon fiber curved plate has a heel longitudinal curve, and the forefoot longitudinal curve and the heel longitudinal curve form the longitudinal arch shape, and the forefoot longitudinal curve includes a first forefoot longitudinal curve section and a second forefoot longitudinal curve section, wherein the first forefoot longitudinal curve section is a straight line section with an inclination angle of 23°-30° relative to a horizontal plane, the second forefoot longitudinal curve section is an arc, and has a arc radius $r_2$ of 70-80 mm and a central angle $\theta_2$ of 50°-60°, the second forefoot longitudinal curve section is located at a tail end of a front part of the forefoot longitudinal curve, and the first forefoot longitudinal curve section and the second forefoot longitudinal curve section are in smooth tangent connection.

In some embodiments, the transverse arch shape of the forefoot carbon fiber curved plate includes a first cross-sectional curve in the rear of the curved plate, a second cross-sectional curve in the middle of the curved plate, and a third cross-sectional curve in the front of the curved plate; and the forefoot carbon fiber curved plate includes a rear arc portion, a middle curved surface portion, a first front portion and a second front portion which are sequentially connected, wherein the first cross-sectional curve is arranged on a connection surface between the rear arc portion and the middle curved surface portion, the second cross-sectional curve is disposed on a connection surface between the middle curved surface portion and the first front portion, the third cross-sectional curve is disposed on a connection surface between the first front portion and the second front potion, and a left foretoe and a right foretoe are arranged on the first front potion and the second front potion.

In some embodiments, the first cross-sectional curve is an arc curve, and has an arc radius $r_1$ of 30-35 mm and a central angle $\theta_1$ of 50°-60°; a mathematical expression of the second cross-sectional curve is: $y = -0.00009x^3 + 0.015x^2 + 0.02x + 31.5$ ; and a mathematical expression of the third cross-sectional curve is:

$$y = -0.000055x^3 + 0.006x^2 + 0.05x + 14.5 \ .$$

The present invention has the beneficial effects:

1. The conventional carbon fiber prosthetic foot straight in the transverse direction is changed, the shape of the transverse curved surface of the prosthetic foot is designed in combination with the three-dimensional curvature features of the longitudinal arch and the transverse arch of the human foot, and a transversely bent carbon fiber plate improves the overall rigidity of the prosthetic foot while reducing the thickness of the carbon fiber plate, so as to achieve better supporting and propelling effects and lighter and more comfortable use experience; and

2. according to the three-dimensional curvature change features of the longitudinal arch and the transverse arch of the human foot, a transverse curvature of the carbon fiber prosthetic foot is designed into gradient change, such that the rigidity distribution of the prosthetic foot is changed in a gradient manner, which better meet the human engineering. Generally, the rigidity distribution of the prosthetic foot is as follows: the rigidity of the rear part is larger, and the rigidity of the front part is smaller; and the rigidity of inner partial toes is larger, and the rigidity of outer partial toes is smaller. The present invention is simple in design structure, but can dynamically meet different requirements on the rigidity of a bearing part and a part, for transmitting ground reaction force, of the prosthetic foot under different road conditions and different movement states, such that the response of the prosthetic foot to the movement is more intelligent, timely and reliable.

**Brief Description of the Drawings**

The accompanying drawings, forming a part of the present application, of the description serve to provide a further understanding of the present invention, and the illustrative embodiments of the present invention and the description of the illustrative embodiments serve to explain the present invention and are not to be construed as unduly limiting the present invention. In the drawings:

Fig. 1 illustrates an axonometric view (in a rear right direction) of a prosthetic foot with a gradient rigidity change and curvature features of a transverse arch of a human body.
Fig. 2 illustrates an axonometric view (in a front right direction) of the prosthetic foot with the gradient rigidity change and the curvature features of the transverse arch of the human body.
Fig. 3 illustrates an axonometric view (in a front right direction) of a forefoot carbon fiber curved plate A.
Fig. 4 illustrates a structural schematic diagram (a front view) of the forefoot carbon fiber curved plate A.
Fig. 5 illustrates a cross-sectional view (in a front right direction) of the forefoot carbon fiber curved plate A.
Fig. 6 illustrates an axonometric view (in a rear right direction) of a heel carbon fiber curved plate B.
Fig. 7 illustrates a structural schematic diagram (a right view) of a forefoot longitudinal curve G and a heel longitudinal curve H.
Fig. 8 illustrates an axonometric view of a connection assembly C.
Fig. 9 illustrates an axonometric view of a square cone joint 6.
Fig. 10 illustrates an axonometric view of an upper connection base 7.
Fig. 11 illustrates an axonometric view of a lower abutting plate 8.
Fig. 12 illustrates an axonometric view of an arrangement relation among the forefoot carbon fiber curved plate A, the heel carbon fiber curved plate B, and the connection assembly C.

In the figures: A. forefoot carbon fiber curved plate B. heel carbon fiber curved plate C. connection assembly D. first cross-sectional curve E. second cross-sectional curve F. third cross-sectional curve G. forefoot longitudinal curve H. heel longitudinal curve 1. first bolt set 2. second bolt set 3. third bolt set 4a. first left hole 4b. first right hole 4c. rear arc portion 4d. middle curved surface portion 4e. left foretoe 4f. right foretoe 5a. second left hole 5b. second right hole 5c. upper arc portion 5d. middle transition portion 5e. lower curved plate portion 4g. first forefoot longitudinal curve section 4h. second forefoot longitudinal curve section 5f. first heel longitudinal curve section 5g. second heel longitudinal curve section 6. square cone joint 6a. stepped hole 6b. square table 7. upper connection base 7a. middle platform 7b. first arc side wing 7c. third left hole 7d. square shallow recessed groove 7e. middle hole 7f. second arc side wing 7g. third right hole 8. lower abutting plate 8a. fourth left hole 8b. fourth right hole

**Detailed Description of the Embodiments**

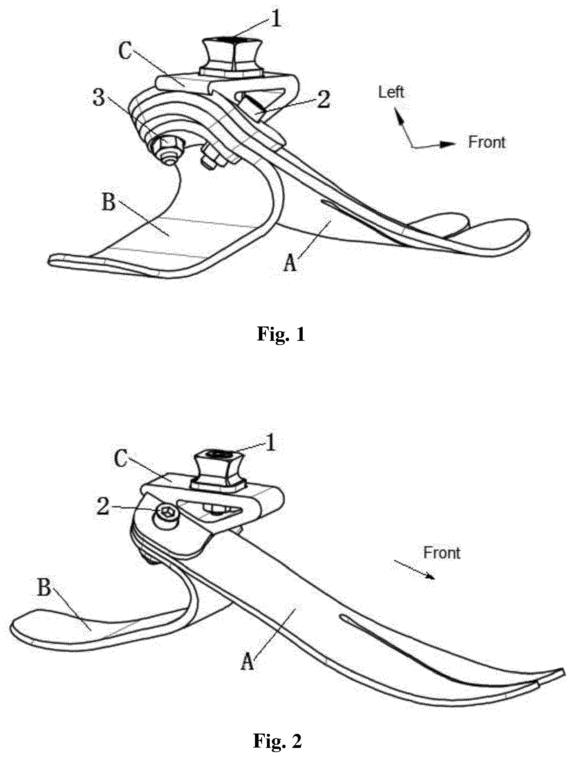

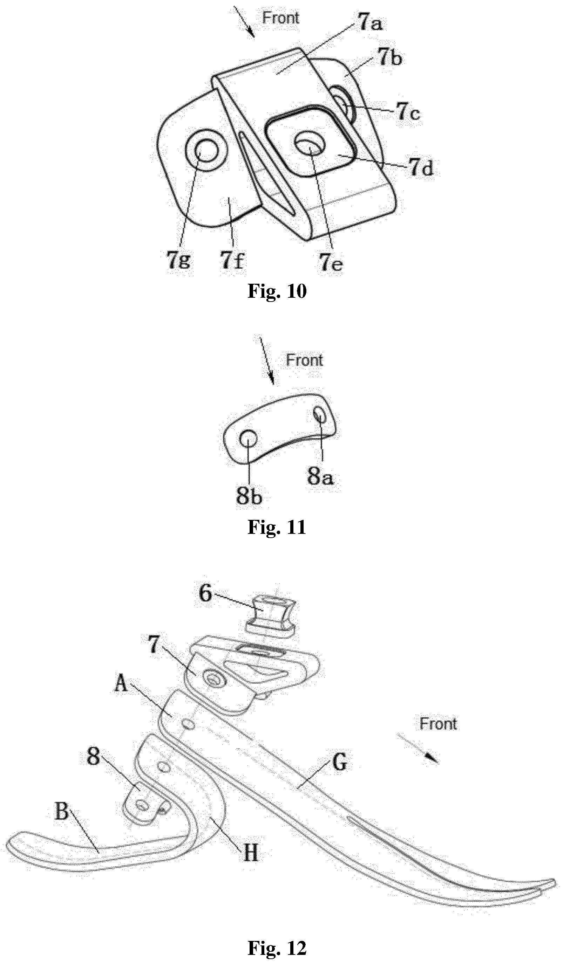

It should be noted that the embodiments of the present application and the features of the embodiments may be combined with one another without conflict. The present invention will be described in detail below with reference to the accompanying drawings and in conjunction with the embodiments.
The present invention will be described hereafter in conjunction with the accompanying drawings.
As shown in Figs. 1, 2 and 12, the present invention includes a forefoot carbon fiber curved plate A, a heel carbon fiber curved plate B, a connection assembly C, a first bolt set 1, a second bolt set 2 and a third bolt set 3, wherein a square cone joint 6 of the connection assembly C, an upper connection base 7 of the connection assembly C, a rear part of the forefoot carbon fiber curved plate A, an upper part of the heel carbon fiber curved plate B and a lower abutting plate 8 of the connection assembly C are sequentially arranged from top to bottom; a square table 6b of the square cone joint 6 in the connection assembly C is arranged in a square shallow recessed groove 7d of the upper connection base 7 and is fixedly connected by the first bolt set 1; a third left hole 7c of a first arc side wing 7b of the upper connection base 7 in the connection assembly C, a first left hole 4a of the forefoot carbon fiber curved plate A, a second left hole 5a of the heel carbon fiber curved plate B and a fourth left hole 8a of the lower abutting plate 8 in the connection assembly C are fixedly connected by the third bolt set 3; a third right hole 7g of a second arc side wing 7f of the upper connection base 7 in the connection assembly C, a first right hole 4b of the forefoot carbon fiber curved plate A, a second right hole 5b of the heel carbon fiber curved plate B and a fourth right hole 8b of the lower abutting plate 8 in the connection assembly C are fixedly connected by the second bolt set 2; and

a forefoot longitudinal curve G of the forefoot carbon fiber curved plate A and a heel longitudinal curve H of the heel carbon fiber curved plate B integrally form a longitudinal arch shape.

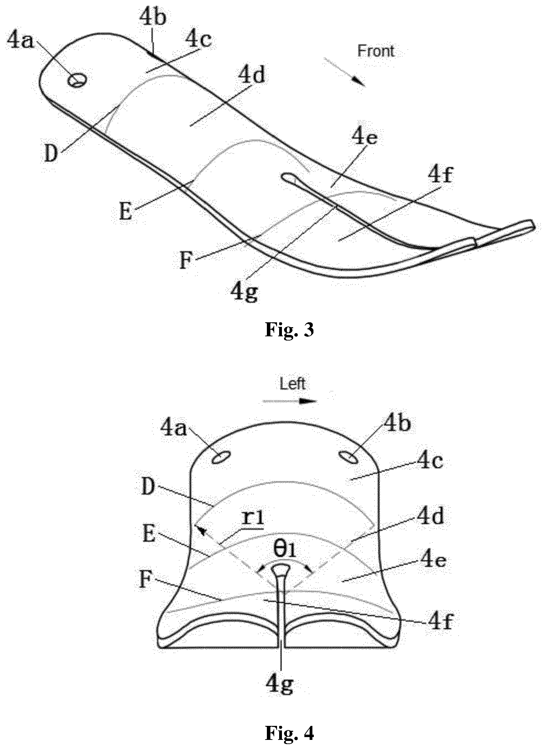

As shown in Figs. 3-5 and 7, the forefoot carbon fiber curved plate A includes a rear arc portion 4c, a middle curved surface portion 4d, a left foretoe 4e, a right foretoe 4f, and the forefoot longitudinal curve G, wherein the first left hole 4a and the first right hole 4b are provided in a rear part of the rear arc portion 4c; a shape of a transverse curved surface of the forefoot carbon fiber curved plate A is determined by a first cross-sectional curve D in a rear of the forefoot carbon fiber curved plate A, a second cross-sectional curve E in a middle of the forefoot carbon fiber curved plate and a third cross-sectional curve F in the front of the forefoot carbon fiber curved plate, wherein the first cross-sectional curve D is an arc curve, and has an arc radius $r_1$ of 30-35 mm and a central angle $\theta_1$ of 50°-60°;

a mathematical expression of the second cross-sectional curve E is:

$$y = -0.00009x^3 + 0.015x^2 + 0.02x + 31.5 \; ;$$

and a mathematical expression of the third cross-sectional curve F is:

$$y = -0.000055x^3 + 0.006x^2 + 0.05x + 14.5 \; .$$

Shapes of the second cross-sectional curve E and the third cross-sectional curve F are drawn according to a basic shape of the transverse arch of the human body, and have a larger curvature of inner sides and a smaller curvature of outer sides; the rear arc portion 4c is an arc cylindrical surface which is controlled by the first cross-sectional curve D and has a thickness of 4-5 mm, and the first left hole 4a and the first right hole 4b are provided in a rear part of the rear arc portion 4c; the middle curved surface portion 4d is a curved surface formed by the first cross-sectional curve D and the second cross-sectional curve E;

an entire front part of the forefoot carbon fiber curved plate A is a curved surface formed by the second cross-sectional curve E and the third cross-sectional curve F, and a bottommost part thereof is an upward straight arc surface and is divided into the left foretoe 4e and the right foretoe 4f by a thin groove 4g in the middle of the front part of the forefoot carbon fiber curved plate A; a thickness from the middle curved surface portion 4d to the top ends of the left foretoe 4e and the right foretoe 4f is uniformly changed, from large to small, from back to front, a maximum thickness is identical to a thickness of the rear arc portion 4c, and a minimum thickness is 2.5 mm; and the forefoot longitudinal curve G includes a first forefoot longitudinal curve section 4g and a second forefoot longitudinal curve section 4h, the first forefoot longitudinal curve section 4g is a straight line section with an inclination angle of 23°-30°, and the second forefoot longitudinal curve section 4h is an arc, having an arc radius $r_2$ of 70-80 mm and a central angle $\theta_2$ of 50°-60°, and is located at a tail end of the front part of the forefoot longitudinal curve G, and the first forefoot longitudinal curve section 4g and the second forefoot longitudinal curve section 4h are in smooth tangent connection.

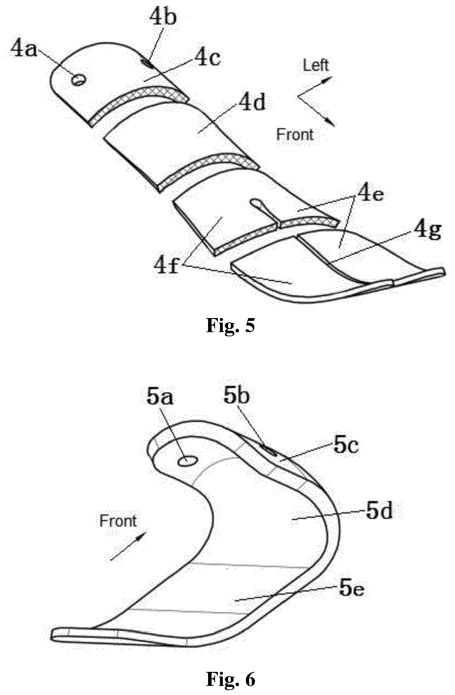

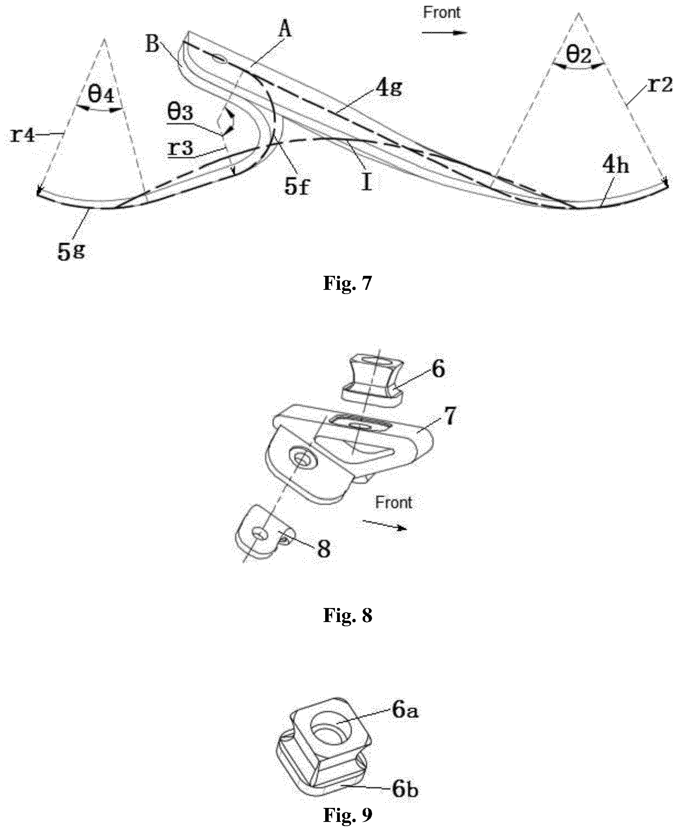

As shown in Figs. 6 and 7, the heel carbon fiber curved plate B includes an upper arc portion 5c, a middle transition portion 5d, a lower curved plate portion 5e and a heel longitudinal curve H, wherein the upper arc portion 5a is an arc plate with transversely distributed curvature and has a shape matched with a first cross-sectional curve D in the forefoot carbon fiber curved plate A; two symmetrical through holes, that is, the second left hole 5a and the second right hole 5b, are provided in a left side and a right side of the upper arc portion 5c; a bottommost part of the lower curved plate portion 5e is an upward transverse straight arc surface; the upper arc portion 5c and the lower curved plate potion 5e are smoothly connected together by the middle transition portion 5d; a thickness of a curved plate of the upper arc portion 5c of the heel carbon fiber curved plate B is uniform and is 5-7 mm; and a thickness of the middle transition portion 5d and a thickness of the lower curved plate part 5e are uniformly changed, from large to small, from back to front, a maximum thickness of each of the middle transition portion 5d and the lower curved plate part 5e is consistent with a thickness of the upper arc portion 5c, and a minimum thickness is 3 mm.

The heel longitudinal curve H includes a first heel longitudinal curve section 5f and a second heel longitudinal curve section 5g, wherein the first heel longitudinal curve section 5f includes an inclined straight line and an arc, wherein the arc has a radius $r_3$ of 70-80 mm and a central angle $\theta_3$ of 130°-150°, and the first heel longitudinal curve section is located on an upper middle part of the heel longitudinal curve H, wherein the second heel longitudinal curve section 5g includes an inclined straight line and an arc, having an arc radius $r_4$ of 60-70 mm and a central angle $\theta_4$ of 8°-12°, and is located on a lower part of the heel longitudinal curve H, and the first heel longitudinal curve section 5f and the second heel longitudinal curve section 5g are in smooth tangent connection by the straight line.

As shown in Figs. 8-11, the connection assembly C includes the square cone joint 6, the upper connection base 7 and the lower abutting plate 8, wherein a lower part of the square cone joint 6 is the square table 6b, and a center of the square cone joint 6 is provided with a stepped hole 6a; the upper connection base 7 includes a middle platform 7a, and the first arc side wing 7b and the second arc side wing 7f which are symmetrically arranged left and right, wherein the middle platform 7a is a hollow triangular pyramid frustum horizontally disposed in a left-right direction, the square shallow recessed groove 7d and a middle hole 7e are provided in a top face of the middle platform 7a, the third left hole 7c is provided in the first arc side wing 7b, and the third right hole 7g is provided in the second arc side wing 7f; the fourth left hole 8a and the fourth right hole 8b are provided in the lower abutting plate 8; and the square cone joint 6, the upper connection base 7 and the lower abutting plate 8 are sequentially arranged from top to bottom.

The present invention provides a prosthetic foot, including a forefoot carbon fiber curved plate A, a heel carbon fiber curved plate B, a connection assembly (C), a first fastening assembly, a second fastening assembly, and a third fastening assembly; a square cone joint 6 of the connection assembly C is disposed in a square shallow recessed groove 7d of an upper connection base 7 and is connected by the first fastening assembly; a first arc side wing 7b of the upper connection base 7 in the connection assembly C, the forefoot carbon fiber curved plate A, the heel carbon fiber curved plate B and a lower abutting plate 8 in the connection assembly C are fixedly connected by the third fastening assembly; a second arc side wing 7f of the upper connection base 7 in the connection assembly C, the forefoot carbon fiber curved plate A, the heel carbon fiber curved plate B and the lower abutting plate 8 in the connection assembly C are fixedly connected by the second fastening assembly; and the forefoot carbon fiber curved plate A and the heel carbon fiber curved plate B form a longitudinal arch shape, and the forefoot carbon fiber curved plate A forms a transverse arch shape.

The longitudinal arch shape refers to an arch-shaped structure which protrudes upwards in a length direction (that is, a front-back direction) of the human foot or the prosthetic foot and has a certain radian, and the transverse arch refers to an arch-shaped structure which protrudes upwards in a width direction (that is, an inner-outer direction) of the human foot or the prosthetic foot and has a certain radian.

In this embodiment, the first fastening assembly is a first bolt set 1, the second fastening assembly is a second bolt set 2, and the third fastening assembly is a third bolt set 3.

In the embodiment, the square cone joint 6 of the connection assembly C, the upper connection base 7 of the connection assembly C, a rear part of the forefoot carbon fiber curved plate A, an upper part of the heel carbon fiber curved plate B and the lower abutting plate 8 of the connection assembly C are sequentially arranged from top to bottom.

In this embodiment, the forefoot carbon fiber curved plate A has a forefoot longitudinal curve G, and the heel carbon fiber curved plate B has a heel longitudinal curve H, and the forefoot longitudinal curve g and the heel longitudinal curve H form the longitudinal arch shape, and the forefoot longitudinal curve G includes a first forefoot longitudinal curve section 4g and a second forefoot longitudinal curve section 4h, wherein the first forefoot longitudinal curve section 4g is a straight line section with an inclination angle of 23°-30° relative to a horizontal plane, the second forefoot longitudinal curve section 4h is an arc, and has a arc radius $r_2$ of 70-80 mm and a central angle $\theta_2$ of 50°-60°, the second forefoot longitudinal curve section 4h is located at a tail end of a front part of the forefoot longitudinal curve G, and the first forefoot longitudinal curve section 4g and the second forefoot longitudinal curve section 4h are in smooth tangent connection.

In the embodiment, the transverse arch shape of the forefoot carbon fiber curved plate A includes a first cross-sectional curve D in the rear of the curved plate, a second cross-sectional curve E in the middle of the curved plate, and a third cross-sectional curve F in the front of the curved plate; and the forefoot carbon fiber curved plate A includes a rear arc portion 4c, a middle curved surface portion 4d, a first front portion and a second front portion which are sequentially connected, wherein the first cross-sectional curve D is arranged on a connection surface between the rear arc portion 4c and the middle curved surface portion 4d, the second cross-sectional curve E is disposed on a connection surface between the middle curved surface portion 4d and the first front portion, the third cross-sectional curve F is disposed on a connection surface between the first front portion and the second front potion, and a left foretoe 4e and a right foretoe 4f are disposed on the first front potion and the second front potion.

In this embodiment, the first cross-sectional curve D is an arc curve, and has an arc radius $r_1$ of 30-35 mm and a central angle $\theta_1$ of 50°-60°; a mathematical expression of the second cross-sectional curve E is: $y = -0.00009x^3 + 0.015x^2 + 0.02x + 31.5$ ; and a mathematical expression of the third cross-sectional curve F is:

$$y = -0.000055x^3 + 0.006x^2 + 0.05x + 14.5 \ .$$

**Claims**

1. A prosthetic foot, comprising a forefoot carbon fiber curved plate (A), a heel carbon fiber curved plate (B), a connection assembly (C), a first fastening assembly, a second fastening assembly, a third fastening assembly, an upper connection base and a square cone joint,

    wherein the forefoot carbon fiber curved plate and the heel carbon fiber curved plate form a longitudinal arch shape, and the forefoot carbon fiber curved plate forms a transverse arch shape **characterized in that**:

the square cone joint (6) of the connection assembly (C) is disposed in a square shallow recessed groove (7d) of the upper connection base (7) in the connection assembly (C) and is connected by the first fastening assembly, a center of the square cone joint (6) is provided with a stepped hole (6a), the first fastening assembly is penetrated in the stepped hole (6a);

a first arc side wing (7b) of the upper connection base (7) in the connection assembly (C), the forefoot carbon fiber curved plate (A), the heel carbon fiber curved plate (B) and a lower abutting plate (8) in the connection assembly (C) are fixedly connected by the third fastening assembly; and

a second arc side wing (7f) of the upper connection base (7) in the connection assembly (C), the forefoot carbon fiber curved plate (A), the heel carbon fiber curved plate (B) and the lower abutting plate (8) in the connection assembly (C) are fixedly connected by the second fastening assembly.

2. The prosthetic foot according to claim 1, wherein said the first fastening assembly, said the second fastening assembly, and said the third fastening assembly are respectively a first bolt set (1), a second bolt set (2) and a third bolt set (3); a square cone joint (6) of the connection assembly (C), an upper connection base (7) of the connection assembly (C), a rear part of the forefoot carbon fiber curved plate (A), an upper part of the heel carbon fiber curved plate (B) and a lower abutting plate (8) of the connection assembly (C) are sequentially arranged from top to bottom; a square table (6b) of the square cone joint (6) is arranged in a square shallow recessed groove (7d) of the upper connection base (7) and is fixedly connected by the first bolt set (1), a center of the square cone joint (6) is provided with a stepped hole (6a), the first bolt set (1) is penetrated in the stepped hole (6a); a third left hole (7c) of a first arc side wing (7b) of the upper connection base (7) in the connection assembly (C), a first left hole (4a) of the forefoot carbon fiber curved plate (A), a second left hole (5a) of the heel carbon fiber curved plate (B) and a fourth left hole (8a) of the lower abutting plate (8) in the connection assembly (C) are fixedly connected by the third bolt set (3); a third right hole (7g) of a second arc side wing (7f) of the upper connection base (7) in the connection assembly (C), a first right hole (4b) of the forefoot carbon fiber curved plate (A), a second right hole (5b) of the heel carbon fiber curved plate (B) and a fourth right hole (8b) of the lower abutting plate (8) in the connection assembly (C) are fixedly connected by the second bolt set (2); and a forefoot longitudinal curve (G) of the forefoot carbon fiber curved plate (A) and a heel longitudinal curve (H) of the heel carbon fiber curved plate (B) integrally form a longitudinal arch shape.

3. The prosthetic foot according to claim 2, wherein the forefoot carbon fiber curved plate (A) comprises a rear arc portion (4c), a middle curved surface portion (4d), a left foretoe (4e), a right foretoe (4f), and the forefoot longitudinal curve (G); wherein the first left hole (4a) and the first right hole (4b) are provided in a rear part of the rear arc portion (4c); a shape of a transverse curved surface of the forefoot carbon fiber curved plate (A) is determined by a first cross-sectional curve (D) in a rear of the forefoot carbon fiber curved plate (A), a second cross-sectional curve (E) in a middle of the forefoot carbon fiber curved plate (A) and a third cross-sectional curve (F) in a front of the forefoot carbon fiber curved plate (A), the first cross-sectional curve (D) is an arc curve, and having an arc radius $r_1$ of 30-35 mm and a central angle $\theta_1$ of 50°-60°;

a mathematical expression of the second cross-sectional curve (E) is:

$$y = -0.00009x^3 + 0.015x^2 + 0.02x + 31.5 \;;$$

a mathematical expression of the third cross-sectional curve (F) is:

$$y = -0.000055x^3 + 0.006x^2 + 0.05x + 14.5 \;;$$

shapes of the second cross-sectional curve (E) and the third cross-sectional curve (F) are drawn according to a basic shape of a transverse arch of the human body, and have a larger curvature of inner sides and a smaller curvature of outer sides; the rear arc portion (4c) is an arc cylindrical surface which is controlled by the first cross-sectional curve (D) and has a thickness of 4-5 mm; the first left hole (4a) and the first right hole (4b) are provided in a rear part of the rear arc portion (4c); the middle curved surface portion (4d) is a curved surface formed by the first cross-sectional curve (D) and the second cross-sectional curve (E); an entire front part of the forefoot carbon fiber curved plate (A) is a curved surface formed by the second cross-sectional curve (E) and the third cross-sectional curve (F), and a bottommost part thereof is an upward straight arc surface and is divided into the left foretoe (4e) and the right foretoe (4f) by a thin groove (41) in a middle of a front part of the forefoot carbon fiber curved plate (A); a thickness from the middle curved surface portion (4d) to top ends of the left foretoe (4e) and the right foretoe (4f) is uniformly changed, from large to small, from back to front, a maximum thickness is identical to a thickness of the rear arc portion (4c), and a minimum thickness is 2.5 mm; and the forefoot longitudinal curve (G) comprises a first forefoot longitudinal curve section (4g) and a second forefoot longitudinal curve section (4h), the first forefoot longitudinal curve section (4g) is a straight line section with an inclination angle of 23°-30°, and the second forefoot longitudinal curve section (4h) is an arc, having an arc radius $r_2$ of 70-80 mm and a central angle $\theta_2$ of 50°-60°, and is located at a tail end of the front part of the forefoot longitudinal curve (G), and the first forefoot longitudinal curve section (4g) and the second forefoot longitudinal curve section (4h) are in smooth tangent connection.

4. The prosthetic foot according to claim 2, wherein the heel carbon fiber curved plate (B) comprises an upper arc portion (5c), a middle transition portion (5d), a lower curved plate portion (5e) and a heel longitudinal curve (H), wherein the upper arc portion (5a) is an arc plate with transversely distributed curvature and has a shape matched with a first cross-sectional curve (D) in the forefoot carbon fiber curved plate (A); two symmetrical through holes, that is, the second left hole (5a) and the second right hole (5b), are provided in a left side and a right side of the upper arc portion (5c); a bottommost part of the lower curved plate portion (5e) is an upward transverse straight arc surface; the upper arc portion (5c) and the lower curved plate potion (5e) are smoothly connected together by the middle transition portion (5d); a thickness of a curved plate of the upper arc portion (5c) of the heel carbon fiber curved plate (B) is uniform and is 5-7 mm; a thickness of the middle transition portion (5d) and a thickness of the lower curved plate part (5e) are uniformly changed, from large to small, from back to front, a maximum thickness of each of the middle transition portion (5d) and the lower curved plate part (5e) is consistent with a thickness of the upper arc portion (5c), and a minimum thickness is 3 mm; and the heel longitudinal curve (H) comprises a first heel longitudinal curve section (5f) and a second heel longitudinal curve section (5g), wherein the first heel longitudinal curve section (5f) comprises an inclined straight line and an arc, has an arc radius $r_3$ of 70-80 mm and a central angle $\theta_3$ of 130°-150°, and is located on an upper middle part of the heel longitudinal curve (H), wherein the second heel longitudinal curve section (5g) comprises an inclined straight line and an arc, having an arc radius $r_4$ of 60-70 mm and a central angle $\theta_4$ of 8°-12°, and is located on a lower part of the heel longitudinal curve (H), and the first heel longitudinal curve section (5f) and the second heel longitudinal curve section (5g) are in smooth tangent connection by the straight line.

5. The prosthetic foot according to claim 2, wherein the connection assembly (C) comprises the square cone joint (6), the upper connection base (7) and the lower abutting plate (8), wherein a lower part of the square cone joint (6) is the square table (6b); the upper connection base (7) comprises a middle platform (7a), and the first arc side wing (7b) and the second arc side wing (7f) which are symmetrically arranged left and right, wherein the middle platform (7a) is a hollow triangular pyramid frustum horizontally disposed in a left-right direction, the square shallow recessed groove (7d) and a middle hole (7e) are provided in a top face of the middle platform (7a), the third left hole (7c) is provided in the first arc side wing (7b), and the third right hole (7g) is provided in the second arc side wing (7f); the fourth left hole (8a) and the fourth right hole (8b) are provided in the lower abutting plate (8); and the square cone joint (6), the upper connection base (7) and the lower abutting plate (8) are sequentially arranged from top to bottom.

6. The prosthetic foot according to claim 1, wherein the square cone joint (6) of the connection assembly (C), the upper connection base (7) of the connection assembly (C), the rear part of the forefoot carbon fiber curved plate (A), the upper part of the heel carbon fiber curved plate (B) and the lower abutting plate (8) of the connection assembly (C) are sequentially arranged from top to bottom.

7. The prosthetic foot according to claim 1, wherein the forefoot carbon fiber curved plate (A) has a forefoot longitudinal curve (G), and the heel carbon fiber curved plate (B) has a heel longitudinal curve (H), wherein the forefoot longitudinal curve (G) and the heel longitudinal curve (H) form the longitudinal arch shape, and the forefoot longitudinal curve (G) comprises a first forefoot longitudinal curve section (4g) and a second forefoot longitudinal curve section (4h), wherein the first forefoot longitudinal curve section (4g) is a straight line section with an inclination angle of 23°-30° relative to a horizontal plane, the second forefoot longitudinal curve section (4h) is an arc and has an arc radius $r_2$ of 70-80 mm and a central angle $\theta_2$ of 50°-60°, the second forefoot longitudinal curve section (4h) is located at a tail end of a front part of the forefoot longitudinal curve (G), and the first forefoot longitudinal curve section (4g) and the second forefoot longitudinal curve section (4h) are in smooth tangent connection.

8. The prosthetic foot according to claim 1, wherein the transverse arch shape of the forefoot carbon fiber curved plate (A) comprises a first cross-sectional curve (D) in the rear of the curved plate, a second cross-sectional curve (E) in the middle of the curved plate, and a third cross-sectional curve (F) in the front of the curved plate; and the forefoot carbon fiber curved plate (A) comprises a rear arc portion (4c), a middle curved surface portion (4d), a first front portion and a second front portion which are sequentially connected, wherein the first cross-sectional curve (D) is arranged on a connection surface between the rear arc portion (4c) and the middle curved surface portion (4d), the second cross-sectional curve (E) is disposed on a connection surface between the middle curved surface portion (4d) and the first front portion, the third cross-sectional curve (F) is disposed on a connection surface between the first front portion and the second front potion, and a left foretoe (4e) and a right foretoe (4f) are disposed on the first front potion and the second front potion.

9. The prosthetic foot according to claim 8, wherein

the first cross-sectional curve (D) is an arc curve, and has an arc radius $r_1$ of 30-35 mm and a central angle $\theta_1$ of 50°-60°;
a mathematical expression of the second cross-sectional curve (E) is:

$$y = -0.00009x^3 + 0.015x^2 + 0.02x + 31.5 \;;$$

and
a mathematical expression of the third cross-sectional curve (F) is:

$$y = -0.000055x^3 + 0.006x^2 + 0.05x + 14.5 \;.$$

10. The prosthetic foot according to claim 9, wherein
shapes of the second cross-sectional curve (E) and the third cross-sectional curve (F) are drawn according to a basic shape of a transverse arch of the human body, and have a larger curvature of inner sides and a smaller curvature of outer sides.

11. The prosthetic foot according to claim 9, wherein
the rear arc portion (4c) is an arc cylindrical surface which is controlled by the first cross-sectional curve (D) and has a thickness of 4-5 mm; the first left hole (4a) and the first right hole (4b) are provided in a rear part of the rear arc portion (4c).

12. The prosthetic foot according to claim 9, wherein
the middle curved surface portion (4d) is a curved surface formed by the first cross-sectional curve (D) and the second cross-sectional curve (E).

13. The prosthetic foot according to claim 9, wherein

an entire front part of the forefoot carbon fiber curved plate (A) is a curved surface formed by the second cross-sectional curve (E) and the third cross-sectional curve (F), and a bottommost part thereof is an upward straight arc surface and is divided into the left foretoe (4e) and the right foretoe (4f) by a thin groove (41) in a middle of a front part of the forefoot carbon fiber curved plate (A);
a thickness from the middle curved surface portion (4d) to top ends of the left foretoe (4e) and the right foretoe (4f) is uniformly changed, from large to small, from back to front, a maximum thickness is identical to a thickness of the rear arc portion (4c), and a minimum thickness is 2.5 mm.

14. The prosthetic foot according to claim 9, wherein
the forefoot longitudinal curve (G) comprises a first forefoot longitudinal curve section (4g) and a second forefoot longitudinal curve section (4h), the first forefoot longitudinal curve section (4g) is a straight line section with an inclination angle of 23°-30°, and the second forefoot longitudinal curve section (4h) is an arc, having an arc radius $r_2$ of 70-80 mm and a central angle $\theta_2$ of 50°-60°, and being located at a tail end of the front part of the forefoot longitudinal curve (G), and the first forefoot longitudinal curve section (4g) and the second forefoot longitudinal curve section (4h) are in smooth tangent connection.

**Patentansprüche**

1.  Fussprothese, umfassend eine gekrümmte Vorderfußkohlefaserplatte (A), eine gekrümmte Fersenkohlefaserplatte (B), eine Verbindungsbaugruppe (C), eine erste Befestigungsbaugruppe, eine zweite Befestigungsbaugruppe, eine dritte Befestigungsbaugruppe, eine obere Verbindungsbasis und ein vierkantiges Konusgelenk, wobei die gekrümmte Vorderfußkohlefaserplatte und die gekrümmte Fersenkohlefaserplatte eine Längswölbungsform bilden und die gekrümmte Vorderfußkohlefaserplatte eine Querwölbungsform bildet, **dadurch gekennzeichnet, dass**:

    das vierkantige Konusgelenk (6) der Verbindungsbaugruppe (C) in einer vierkantigen flachen vertieften Rille (7d) der oberen Verbindungsbasis (7) in der Verbindungsbaugruppe (C) angeordnet ist und durch die erste Befestigungsbaugruppe verbunden ist, ein Zentrum des vierkantigen Konusgelenks (6) mit einem abgestuften Loch (6a) versehen ist, die erste Befestigungsbaugruppe im abgestuften Loch (6a) durchdrungen ist;
    ein erster Bogenseitenflügel (7b) der oberen Verbindungsbasis (7) in der Verbindungsbaugruppe (C), die gekrümmte Vorderfußkohlefaserplatte (A), die gekrümmte Fersenkohlefaserplatte (B) und eine untere Anlageplatte (8) in der Verbindungsbaugruppe (C) sind durch die dritte Befestigungsbaugruppe fest verbunden; und
    ein zweiter Bogenseitenflügel (7f) der oberen Verbindungsbasis (7) in der Verbindungsbaugruppe (C), die gekrümmte Vorderfußkohlefaserplatte (A), die gekrümmte Fersenkohlefaserplatte (B) und die untere Anlageplatte (8) in der Verbindungsbaugruppe (C) sind durch die zweite Befestigungsbaugruppe fest verbunden.

2.  Fussprothese nach Anspruch 1, wobei die erste Befestigungsbaugruppe, die zweite Befestigungsbaugruppe und die dritte Befestigungsbaugruppe jeweils ein erster Bolzensatz (1), ein zweiter Bolzensatz (2) und ein dritter Bolzensatz (3) sind; ein vierkantiges Konusgelenk (6) der Verbindungsbaugruppe (C), eine obere Verbindungsbasis (7) der Verbindungsbaugruppe (C), ein hinterer Teil der gekrümmten Vorderfußkohlefaserplatte (A), ein oberer Teil der gekrümmten Fersenkohlefaserplatte (B) und eine untere Anlageplatte (8) der Verbindungsbaugruppe (C) sind sequentiell von oben nach unten angeordnet;
    eine vierkantige Tafel (6b) des vierkantigen Konusgelenks (6) ist in einer vierkantigen flachen vertieften Rille (7d) der oberen Verbindungsbasis (7) angeordnet und ist durch den ersten Bolzensatz (1) fest verbunden, ein Zentrum des vierkantigen Konusgelenks (6) ist mit einem abgestuften Loch (6a) versehen, der erste Bolzensatz (1) ist im abgestuften Loch (6a) durchdrungen; ein drittes linkes Loch (7c) eines ersten Bogenseitenflügels (7b) der oberen Verbindungsbasis (7) in der Verbindungsbaugruppe (C), ein erstes linkes Loch (4a) der gekrümmten Vorderfußkohlefaserplatte (A), ein zweites linkes Loch (5a) der gekrümmten Fersenkohlefaserplatte (B) und ein viertes linkes Loch (8a) der unteren Anlageplatte (8) in der Verbindungsbaugruppe (C) sind durch den dritten Bolzensatz (3) fest verbunden; ein drittes rechtes Loch (7g) eines zweiten Bogenseitenflügels (7f) der oberen Verbindungsbasis (7) in der Verbindungsbaugruppe (C), ein erstes rechtes Loch (4b) der gekrümmten Vorderfußkohlefaserplatte (A), ein zweites rechtes Loch (5b) der gekrümmten Fersenkohlefaserplatte (B) und ein viertes rechtes Loch (8b) der unteren Anlageplatte (8) in der Verbindungsbaugruppe (C) sind durch den zweiten Bolzensatz (2) fest verbunden; und eine Vorderfußlängskurve (G) der gekrümmten Vorderfußkohlefaserplatte (A) und eine Fersenlängskurve (H) der gekrümmten Fersenkohlefaserplatte (B) bilden einstückig eine Längswölbungsform.

3.  Fussprothese nach Anspruch 2, wobei die gekrümmte Vorderfußkohlefaserplatte (A) einen hinteren Bogenabschnitt (4c), einen mittleren gekrümmten Oberflächenabschnitt (4d), einen linken Vorderzeh (4e), einen rechten Vorderzeh (4f) und die Vorderfußlängskurve (G) umfasst; wobei das erste linke Loch (4a) und das erste rechte Loch (4b) in einem hinteren Teil des hinteren Bogenabschnitts (4c) bereitgestellt sind; eine Form einer quer gekrümmten Oberfläche der gekrümmten Vorderfußkohlefaserplatte (A) wird durch eine erste Querschnittskurve (D) in einer Rückseite der gekrümmten Vorderfußkohlefaserplatte (A), eine zweite Querschnittskurve (E) in einer Mitte der gekrümmten Vorderfußkohlefaserplatte (A) und eine dritte Querschnittskurve (F) in einer Vorderseite der gekrümmten Vorderfußkohlefaserplatte (A) bestimmt, die erste Querschnittskurve (D) ist eine Bogenkurve und aufweisend einen Bogenradius $r_1$ von 30-35 mm und einen Zentriwinkel $\theta_1$ von 50°-60°;

    ein mathematischer Ausdruck der zweiten Querschnittskurve (E) ist:

    $$y = -0{,}00009x^3 + 0{,}015x^2 + 0{,}02x + 31{,}5;$$

    ein mathematischer Ausdruck der dritten Querschnittskurve (F) ist:

    $$y = -0{,}000055x^3 + 0{,}006x^2 + 0{,}05x + 14{,}5;$$

Formen der zweiten Querschnittskurve (E) und der dritten Querschnittskurve (F) werden gemäß einer Grundform einer Querwölbung des menschlichen Körpers gezeichnet und weisen eine größere Krümmung der Innenseiten und eine kleinere Krümmung der Außenseiten auf; der hintere Bogenabschnitt (4c) ist eine zylindrische Bogenoberfläche, die durch die erste Querschnittskurve (D) kontrolliert wird und eine Dicke von 4-5 mm aufweist; das erste linke Loch (4a) und das erste rechte Loch (4b) sind in einem hinteren Teil des hinteren Bogenabschnitts (4c) bereitgestellt; der mittlere gekrümmte Oberflächenabschnitt (4d) ist eine gekrümmte Oberfläche, die durch die erste Querschnittskurve (D) und die zweite Querschnittskurve (E) gebildet wird; ein gesamter vorderer Teil der gekrümmten Vorderfußkohlefaserplatte (A) ist eine gekrümmte Oberfläche, die durch die zweite Querschnittskurve (E) und die dritte Querschnittskurve (F) gebildet ist, und ein unterster Teil davon ist eine nach oben gerichtete gerade Bogenoberfläche und ist durch eine dünne Rille (41) in einer Mitte eines vorderen Teils der gekrümmten Vorderfußkohlefaserplatte (A) in den linken Vorderzeh (4e) und in den rechten Vorderzeh (4f) unterteilt; eine Dicke vom mittleren gekrümmten Oberflächenabschnitt (4d) bis zu den oberseitigen Enden des linken Vorderzehs (4e) und des rechten Vorderzehs (4f) wird gleichmäßig von groß zu klein von hinten nach vorne geändert, eine maximale Dicke ist mit einer Dicke des hinteren Bogenabschnitts (4c) identisch und eine minimale Dicke beträgt 2,5 mm; und die Vorderfußlängskurve (G) umfasst ein erstes Vorderfußlängskurventeilstück (4g) und ein zweites Vorderfußlängskurventeilstück (4h), das erste Vorderfußlängskurventeilstück (4g) ist ein geradliniges Teilstück mit einem Neigungswinkel von 23°-30° und das zweite Vorderfußlängskurventeilstück (4h) ist ein Bogen, aufweisend einen Bogenradius $r_2$ von 70-80 mm und einen Zentriwinkel $\theta_2$ von 50°-60° und befindet sich an einem Schwanzende des vorderen Teils der Vorderfußlängskurve (G) und das erste Vorderfußlängskurventeilstück (4g) und das zweite Vorderfußlängskurventeilstück (4h) stehen in einer glatten tangentialen Verbindung.

4.  Fussprothese nach Anspruch 2, wobei die gekrümmte Fersenkohlefaserplatte (B) einen oberen Bogenabschnitt (5c), einen mittleren Übergangsabschnitt (5d), einen unteren gekrümmten Plattenabschnitt (5e) und eine Fersenlängskurve (H) umfasst, wobei der obere Bogenabschnitt (5a) eine Bogenplatte mit quer verteilter Krümmung ist und eine Form aufweist, die an eine erste Querschnittskurve (D) in der gekrümmten Vorderfußkohlefaserplatte (A) angepasst ist; zwei symmetrische Durchgangslöcher, d. h. das zweite linke Loch (5a) und das zweite rechte Loch (5b), sind in einer linken Seite und einer rechten Seite des oberen Bogenabschnitts (5c) bereitgestellt; ein unterster Teil des unteren gekrümmten Plattenabschnitts (5e) ist eine nach oben gerichtete gerade Querbogenoberfläche; der obere Bogenabschnitt (5c) und der untere gekrümmte Plattenabschnitt (5e) sind durch den mittleren Übergangsabschnitt (5d) glatt miteinander verbunden; eine Dicke einer gekrümmten Platte des oberen Bogenabschnitts (5c) der gekrümmten Fersenkohlefaserplatte (B) ist gleichmäßig und beträgt 5-7 mm; eine Dicke des mittleren Übergangsabschnitts (5d) und eine Dicke des unteren gekrümmten Plattenteils (5e) werden gleichmäßig von groß zu klein von hinten nach vorne geändert, eine maximale Dicke eines jeden des mittleren Übergangsabschnitts (5d) und des unteren gekrümmten Plattenteils (5e) stimmt mit einer Dicke des oberen Bogenabschnitts (5c) überein und eine minimale Dicke beträgt 3 mm; und die Fersenlängskurve (H) umfasst ein erstes Fersenlängskurventeilstück (5f) und ein zweites Fersenlängskurventeilstück (5g), wobei das erste Fersenlängskurventeilstück (5f) eine geneigte gerade Linie und einen Bogen umfasst, einen Bogenradius $r_3$ von 70-80 mm und einen Zentriwinkel $\theta_3$ von 130°-150° aufweist und sich auf einem oberen Mittelteil der Fersenlängskurve (H) befindet, wobei das zweite Fersenlängskurventeilstück (5g) eine geneigte gerade Linie und einen Bogen umfasst, aufweisend einen Bogenradius $r_4$ von 60-70 mm und einen Zentriwinkel $\theta_4$ von 8°-12° und sich an einem unteren Teil der Fersenlängskurve (H) befindet und das erste Fersenlängskurventeilstück (5f) und das zweite Fersenlängskurventeilstück (5g) durch die gerade Linie in glatter tangentialer Verbindung stehen.

5.  Fussprothese nach Anspruch 2, wobei die Verbindungsbaugruppe (C) das vierkantige Konusgelenk (6), die obere Verbindungsbasis (7) und die untere Anlageplatte (8) umfasst, wobei ein unterer Teil des vierkantigen Konusgelenks (6) die vierkantige Tafel (6b) ist; die obere Verbindungsbasis (7) umfasst eine mittlere Plattform (7a) und den ersten Bogenseitenflügel (7b) und den zweiten Bogenseitenflügel (7f), die symmetrisch links und rechts angeordnet sind, wobei die mittlere Plattform (7a) ein hohler dreieckiger Pyramidenstumpf ist, der horizontal in einer Links-Rechts-Richtung angeordnet ist, die vierkantige flache vertiefte Rille (7d) und ein mittleres Loch (7e) in einer oberseitigen Fläche der mittleren Plattform (7a) bereitgestellt sind, das dritte linke Loch (7c) in dem ersten Bogenseitenflügel (7b) bereitgestellt ist und das dritte rechte Loch (7g) in dem zweiten Bogenseitenflügel (7f) bereitgestellt ist; das vierte linke Loch (8a) und das vierte rechte Loch (8b) sind in der unteren Anlageplatte (8) bereitgestellt; und das vierkantige Konusgelenk (6), die obere Verbindungsbasis (7) und die untere Anlageplatte (8) sind sequentiell von oben nach unten angeordnet.

6.  Fussprothese nach Anspruch 1, wobei das vierkantige Konusgelenk (6) der Verbindungsbaugruppe (C), die obere Verbindungsbasis (7) der Verbindungsbaugruppe (C), der hintere Teil der gekrümmten Vorderfußkohlefaserplatte (A), der obere Teil der gekrümmten Fersenkohlefaserplatte (B) und die untere Anlageplatte (8) der Verbindungsbaugruppe (C) sequentiell von oben nach unten angeordnet sind.

7. Fussprothese nach Anspruch 1, wobei die gekrümmte Vorderfußkohlefaserplatte (A) eine Vorderfußlängskurve (G) aufweist und die gekrümmte Fersenkohlefaserplatte (B) eine Fersenlängskurve (H) aufweist, wobei die Vorderfußlängskurve (G) und die Fersenlängskurve (H) die Längswölbungsform bilden und die Vorderfußlängskurve (G) ein erstes Vorderfußlängskurventeilstück (4g) und ein zweites Vorderfußlängskurventeilstück (4h) umfasst, wobei das erste Vorderfußlängskurventeilstück (4g) ein geradliniges Teilstück mit einem Neigungswinkel von 23°-30° relativ zu einer horizontalen Ebene ist, das zweite Vorderfußlängskurventeilstück (4h) ein Bogen ist und einen Bogenradius $r_2$ von 70-80mm und einen Zentriwinkel $\theta_2$ von 50°-60° aufweist, das zweite Vorderfußlängskurventeilstück (4h) sich an einem Schwanzende eines vorderen Teils der Vorderfußlängskurve (G) befindet und das erste Vorderfußlängskurventeilstück (4g) und das zweite Vorderfußlängskurventeilstück (4h) in einer glatten tangentialen Verbindung stehen.

8. Fussprothese nach Anspruch 1, wobei die Querwölbungsform der gekrümmten Vorderfußkohlefaserplatte (A) eine erste Querschnittskurve (D) in der Rückseite der gekrümmten Platte, eine zweite Querschnittskurve (E) in der Mitte der gekrümmten Platte und eine dritte Querschnittskurve (F) in der Vorderseite der gekrümmten Platte umfasst; und die gekrümmte Vorderfußkohlefaserplatte (A) umfasst einen hinteren Bogenabschnitt (4c), einen mittleren gekrümmten Oberflächenabschnitt (4d), einen ersten vorderen Abschnitt und einen zweiten vorderen Abschnitt, die sequentiell verbunden sind, wobei die erste Querschnittskurve (D) auf einer Verbindungsoberfläche zwischen dem hinteren Bogenabschnitt (4c) und dem mittleren gekrümmten Oberflächenabschnitt (4d) angeordnet ist, die zweite Querschnittskurve (E) auf einer Verbindungsoberfläche zwischen dem mittleren gekrümmten Oberflächenabschnitt (4d) und dem ersten vorderen Abschnitt angeordnet ist, die dritte Querschnittskurve (F) auf einer Verbindungsoberfläche zwischen dem ersten vorderen Abschnitt und dem zweiten vorderen Abschnitt angeordnet ist und ein linker Vorderzeh (4e) und ein rechter Vorderzeh (4f) auf dem ersten vorderen Abschnitt und dem zweiten vorderen Abschnitt angeordnet sind.

9. Fussprothese nach Anspruch 8, wobei die erste Querschnittskurve (D) eine Bogenkurve ist und einen Bogenradius $r_1$ von 30-35 mm und einen Zentriwinkel $\theta_1$ von 50°-60° aufweist;

ein mathematischer Ausdruck der zweiten Querschnittskurve (E) ist:

$$y = -0{,}00009x^3 + 0{,}015x^2 + 0{,}02x\ 31{,}5;$$

und
ein mathematischer Ausdruck der dritten Querschnittskurve (F) ist:

$$y = -0{,}000055x^3 + 0{,}006x^2 + 0{,}05x + 14{,}5.$$

10. Fussprothese nach Anspruch 9, wobei Formen der zweiten Querschnittskurve (E) und der dritten Querschnittskurve (F) gemäß einer Grundform einer Querwölbung des menschlichen Körpers gezeichnet sind und eine größere Krümmung der Innenseiten und eine kleinere Krümmung der Außenseiten aufweisen.

11. Fussprothese nach Anspruch 9, wobei der hintere Bogenabschnitt (4c) eine zylindrische Bogenoberfläche ist, die durch die erste Querschnittskurve (D) kontrolliert wird und eine Dicke von 4-5 mm aufweist; wobei das erste linke Loch (4a) und das erste rechte Loch (4b) in einem hinteren Teil des hinteren Bogenabschnitts (4c) bereitgestellt sind.

12. Fussprothese nach Anspruch 9, wobei der mittlere gekrümmte Oberflächenabschnitt (4d) eine gekrümmte Oberfläche ist, die durch die erste Querschnittskurve (D) und die zweite Querschnittskurve (E) gebildet wird.

13. Fussprothese nach Anspruch 9, wobei ein gesamter vorderer Teil der gekrümmten Vorderfußkohlefaserplatte (A) eine gekrümmte Oberfläche ist, die durch die zweite Querschnittskurve (E) und die dritte Querschnittskurve (F) gebildet wird, und ein unterster Teil davon eine nach oben gerichtete gerade Bogenoberfläche ist und durch eine dünne Rille (41) in einer Mitte eines vorderen Teils der gekrümmten Vorderfußkohlefaserplatte (A) in den linken Vorderzeh (4e) und den rechten Vorderzeh (4f) unterteilt ist; eine Dicke vom mittleren gekrümmten Oberflächenabschnitt (4d) bis zu den oberseitigen Enden des linken Vorderzehs (4e) und des rechten Vorderzehs (4f) wird gleichmäßig von groß zu klein von hinten nach vorne geändert, eine maximale Dicke ist mit einer Dicke des hinteren Bogenabschnitts (4c) identisch und eine minimale Dicke beträgt 2,5 mm.

14. Fussprothese nach Anspruch 9, wobei die Vorderfußlängskurve (G) ein erstes Vorderfußlängskurventeilstück (4g) und ein zweites Vorderfußlängskurventeilstück (4h) umfasst, das erste Vorderfußlängskurventeilstück (4g) ein geradliniges Teilstück mit einem Neigungswinkel von 23°-30° ist und das zweite Vorderfußlängskurventeilstück (4h) ein Bogen ist, aufweisend einen Bogenradius $r_2$ von 70-80 mm und einen Zentriwinkel $\theta_2$ von 50°-60° und sich an einem Schwanzende des vorderen Teils der Vorfußlängskurve (G) befindet, und das erste Vorfußlängskurventeilstück (4g) und das zweite Vorfußlängskurventeilstück (4h) in einer glatten tangentialen Verbindung stehen.

**Revendications**

1. Pied prothétique, comprenant une plaque incurvée en fibre de carbone d'avant-pied (A), une plaque incurvée en fibre de carbone de talon (B), un assemblage de raccordement (C), un premier assemblage de fixation, un deuxième assemblage de fixation, un troisième assemblage de fixation, une base de raccordement supérieure et un joint à cône carré, dans lequel la plaque incurvée en fibre de carbone d'avant-pied et la plaque incurvée en fibre de carbone de talon forment une cambrure longitudinale, et la plaque incurvée en fibre de carbone d'avant-pied forme une cambrure transversale, **caractérisé en ce que** :

   le joint à cône carré (6) de l'assemblage de raccordement (C) est disposé dans une rainure renfoncée carrée peu profonde (7d) de la base de raccordement supérieure (7) dans l'assemblage de raccordement (C) et est raccordé par le premier assemblage de fixation, un centre du joint à cône carré (6) est pourvu d'un trou étagé (6a), le premier assemblage de fixation est pénétré dans le trou étagé (6a) ;
   une première aile latérale en arc (7b) de la base de raccordement supérieure (7) dans l'assemblage de raccordement (C), la plaque incurvée en fibre de carbone d'avant-pied (A), la plaque incurvée en fibre de carbone de talon (B) et une plaque de butée inférieure (8) dans l'assemblage de raccordement (C) sont reliées de manière fixe par le troisième assemblage de fixation ; et
   une deuxième aile latérale en arc (7f) de la base de raccordement supérieure (7) dans l'assemblage de raccordement (C), la plaque incurvée en fibre de carbone d'avant-pied (A), la plaque incurvée en fibre de carbone de talon (B) et la plaque de butée inférieure (8) dans l'assemblage de raccordement (C) sont reliées de manière fixe par le deuxième assemblage de fixation.

2. Pied prothétique selon la revendication 1, dans lequel ledit premier assemblage de fixation, ledit deuxième assemblage de fixation et ledit troisième assemblage de fixation sont respectivement un premier ensemble de boulons (1), un deuxième ensemble de boulons (2) et un troisième ensemble de boulons (3) ; un joint à cône carré (6) de l'assemblage de raccordement (C), une base de raccordement supérieure (7) de l'assemblage de raccordement (C), une partie arrière de la plaque incurvée en fibre de carbone d'avant-pied (A), une partie supérieure de la plaque incurvée en fibre de carbone de talon (B) et une plaque de butée inférieure (8) de l'assemblage de raccordement (C) sont disposés séquentiellement de haut en bas ; un plateau carré (6b) du joint à cône carré (6) est disposé dans une rainure renfoncée carrée peu profonde (7d) de la base de raccordement supérieure (7) et est relié de manière fixe par le premier ensemble de boulons (1), un centre du joint à cône carré (6) est pourvu d'un trou étagé (6a), le premier ensemble de boulons (1) est pénétré dans le trou étagé (6a) ; un troisième trou gauche (7c) d'une première aile latérale en arc (7b) de la base de raccordement supérieure (7) dans l'assemblage de raccordement (C), un premier trou gauche (4a) de la plaque incurvée en fibre de carbone d'avant-pied (A), un deuxième trou gauche (5a) de la plaque incurvée en fibre de carbone de talon (B) et un quatrième trou gauche (8a) de la plaque de butée inférieure (8) dans l'assemblage de raccordement (C) sont reliés de manière fixe par le troisième ensemble de boulons (3) ; un troisième trou droit (7g) d'une deuxième aile latérale en arc (7f) de la base de raccordement supérieure (7) dans l'assemblage de raccordement (C), un premier trou droit (4b) de la plaque incurvée en fibre de carbone d'avant-pied (A), un deuxième trou droit (5b) de la plaque incurvée en fibre de carbone de talon (B) et un quatrième trou droit (8b) de la plaque de butée inférieure (8) dans l'assemblage de raccordement (C) sont reliés de manière fixe par le deuxième ensemble de boulons (2) ; et une courbe longitudinale (G) d'avant-pied de la plaque incurvée en fibre de carbone d'avant-pied (A) et une courbe longitudinale (H) de talon de la plaque incurvée en fibre de carbone de talon (B) forment intégralement une cambrure longitudinale.

3. Pied prothétique selon la revendication 2, dans lequel la plaque incurvée en fibre de carbone d'avant-pied (A) comprend une portion d'arc arrière (4c), une portion de surface incurvée médiane (4d), un avant-orteil gauche (4e), un avant-orteil droit (4f), et la courbe longitudinale (G) d'avant-pied ; dans lequel le premier trou gauche (4a) et le premier trou droit (4b) sont prévus dans une partie arrière de la portion d'arc arrière (4c) ; une forme d'une surface incurvée transversale de la plaque incurvée en fibre de carbone d'avant-pied (A) est déterminée par une première courbe à section transversale (D) à l'arrière de la plaque incurvée en fibre de carbone d'avant-pied (A), une deuxième courbe à section transversale (E) au milieu de la plaque incurvée en fibre de carbone d'avant-pied (A) et une troisième courbe à section transversale (F) à l'avant de la plaque incurvée en fibre de carbone d'avant-pied (A), la première courbe à section transversale (D) est une courbe en arc, et ayant un rayon d'arc $r_1$ de 30-35 mm et un angle central $\theta_1$ de 50°-60° ; une expression mathématique de la deuxième courbe à section transversale (E) est :

$$y = -0,00009x^3 + 0,015x^2 + 0,02x + 31,5 ;$$

une expression mathématique de la troisième courbe à section transversale (F) est :

$$y = -0,000055x^3 + 0,006x^2 + 0,05x + 14,5 ;$$

des formes de la deuxième courbe à section transversale (E) et de la troisième courbe à section transversale (F) sont dessinées selon une forme de base d'un arc transversal du corps humain, et ont une plus grande courbure des côtés intérieurs et une plus petite courbure des côtés extérieurs ; la portion d'arc arrière (4c) est une surface cylindrique en arc qui est commandée par la première courbe à section transversale (D) et a une épaisseur de 4-5 mm ; le premier trou gauche (4a) et le premier trou droit (4b) sont prévus dans une partie arrière de la portion d'arc arrière (4c) ; la portion médiane de la surface incurvée (4d) est une surface incurvée formée par la première courbe à section transversale (D) et la deuxième courbe à section transversale (E) ; toute la partie avant de la plaque incurvée en fibre de carbone d'avant-pied (A) est une surface incurvée formée par la deuxième courbe à section transversale (E) et la troisième courbe à section transversale (F), et une partie la plus basse de celle-ci est une surface en arc rectiligne vers le haut et est divisée en l'avant-orteil gauche (4e) et l'avant-orteil droit (4f) par une fine rainure (41) au milieu d'une partie avant de la plaque incurvée en fibre de carbone d'avant-pied (A) ; une épaisseur de la portion médiane de la surface incurvée (4d) aux extrémités supérieures de l'avant-orteil gauche (4e) et de l'avant-orteil droit (4f) est uniformément modifiée, de grande à petite, de l'arrière vers l'avant, une épaisseur maximale est identique à une épaisseur de la portion d'arc arrière (4c), et une épaisseur minimale est de 2,5 mm ; et la courbe longitudinale (G) d'avant-pied comprend une première section de courbe longitudinale (4g) d'avant-pied et une deuxième section de courbe longitudinale (4h) d'avant-pied, la première section de courbe longitudinale (4g) d'avant-pied est une section de ligne rectiligne avec un angle d'inclinaison de 23°-30°, et la deuxième section de courbe longitudinale (4h) d'avant-pied est un arc, ayant un rayon d'arc $r_2$ de 70-80 mm et un angle central $\theta_2$ de 50°-60°, et est située à une extrémité de queue de la partie avant de la courbe longitudinale (G) d'avant-pied, et la première section de courbe longitudinale (4g) d'avant-pied et la deuxième section de courbe longitudinale (4h) d'avant-pied sont en raccordement tangent fluide.

**4.** Pied prothétique selon la revendication 2, dans lequel la plaque incurvée en fibre de carbone de talon (B) comprend une portion d'arc supérieure (5c), une portion de transition médiane (5d), une portion de plaque incurvée inférieure (5e) et une courbe longitudinale (H) de talon, dans lequel la portion d'arc supérieure (5a) est une plaque d'arc ayant une courbure répartie transversalement et une forme correspondant à une première courbe à section transversale (D) dans la plaque incurvée en fibre de carbone d'avant-pied (A) ; deux trous traversants symétriques, à savoir le deuxième trou gauche (5a) et le deuxième trou droit (5b), sont prévus sur un côté gauche et un côté droit de la portion d'arc supérieure (5c) ; une partie la plus basse de la portion de plaque incurvée inférieure (5e) est une surface en arc rectiligne transversale vers le haut ; la portion d'arc supérieure (5c) et la portion de plaque incurvée inférieure (5e) sont reliées de façon fluide par la portion de transition médiane (5d) ; une épaisseur d'une plaque incurvée de la portion d'arc supérieure (5c) de la plaque incurvée en fibre de carbone de talon (B) est uniforme et est de 5-7 mm ; une épaisseur de la portion de transition médiane (5d) et une épaisseur de la partie de plaque incurvée inférieure (5e) sont uniformément modifiées, de grande à petite, de l'arrière vers l'avant, une épaisseur maximale de la portion de transition médiane (5d) et de la partie de plaque incurvée inférieure (5e) correspond à une épaisseur de la portion d'arc supérieure (5c), et une épaisseur minimale est de 3 mm ; et la courbe longitudinale (H) de talon comprend une première section de courbe longitudinale (5f) de talon et une deuxième section de courbe longitudinale (5g) de talon, dans lequel la première section de courbe longitudinale (5f) de talon comprend une ligne rectiligne inclinée et un arc, a un rayon d'arc $r_3$ de 70-80 mm et un angle central $\theta_3$ de 130°-150°, et est située sur une partie médiane supérieure de la courbe longitudinale (H) de talon, dans lequel la deuxième section de courbe longitudinale (5g) de talon comprend une ligne rectiligne inclinée et un arc, ayant un rayon d'arc $r_4$ de 60-70 mm et un angle central $\theta_4$ de 8°-12°, et est située sur une partie inférieure de la courbe longitudinale (H) de talon, et la première section de courbe longitudinale (5f) de talon et la deuxième section de courbe longitudinale (5g) de talon sont reliées par un raccordement tangent fluide par la ligne rectiligne.

5. Pied prothétique selon la revendication 2, dans lequel l'assemblage de raccordement (C) comprend le joint à cône carré (6), la base de raccordement supérieure (7) et la plaque de butée inférieure (8), dans lequel une partie inférieure du joint à cône carré (6) est le plateau carré (6b) ; la base de raccordement supérieure (7) comprend une plateforme médiane (7a), la première aile latérale en arc (7b) et la deuxième aile latérale en arc (7f) qui sont disposées symétriquement à gauche et à droite, dans lequel la plateforme médiane (7a) est un tronc de cône de pyramide triangulaire creux disposé horizontalement dans un sens gauche-droite, la rainure renfoncée carrée peu profonde (7d) et un trou médian (7e) sont prévus dans une face supérieure de la plateforme médiane (7a), le troisième trou gauche (7c) est prévu dans la première aile latérale en arc (7b), et le troisième trou droit (7g) est prévu dans la deuxième aile latérale en arc (7f) ; le quatrième trou gauche (8a) et le quatrième trou droit (8b) sont prévus dans la plaque de butée inférieure (8) ; et le joint à cône carré (6), la base de raccordement supérieure (7) et la plaque de butée inférieure (8) sont disposés séquentiellement de haut en bas.

6. Pied prothétique selon la revendication 1, dans lequel le joint à cône carré (6) de l'assemblage de raccordement (C), la base de raccordement supérieure (7) de l'assemblage de raccordement (C), la partie arrière de la plaque incurvée en fibre de carbone d'avant-pied (A), la partie supérieure de la plaque incurvée en fibre de carbone de talon (B) et la plaque de butée inférieure (8) de l'assemblage de raccordement (C) sont disposés séquentiellement de haut en bas.

7. Pied prothétique selon la revendication 1, dans lequel la plaque incurvée en fibre de carbone d'avant-pied (A) a une courbe longitudinale (G) d'avant-pied, et la plaque incurvée en fibre de carbone de talon (B) a une courbe longitudinale (H) de talon, dans lequel la courbe longitudinale (G) d'avant-pied et la courbe longitudinale (H) de talon forment la cambrure longitudinal, et la courbe longitudinale (G) d'avant-pied comprend une première section de courbe longitudinale (4g) d'avant-pied et une deuxième section de courbe longitudinale (4h) d'avant-pied, dans lequel la première section de courbe longitudinale (4g) d'avant-pied est une section de ligne rectiligne avec un angle d'inclinaison de 23°-30° par rapport à un plan horizontal, la deuxième section de courbe longitudinale (4h) d'avant-pied est un arc et a un rayon d'arc $r_2$ de 70-80 mm et un angle central $\theta_2$ de 50°-60°, la deuxième section de courbe longitudinale (4h) d'avant-pied est située à une extrémité de queue d'une partie avant de la courbe longitudinale (G) d'avant-pied, et la première section de courbe longitudinale (4g) d'avant-pied et la deuxième section de courbe longitudinale (4h) d'avant-pied sont en raccordement tangent fluide.

8. Pied prothétique selon la revendication 1, dans lequel la cambrure transversale de la plaque incurvée en fibre de carbone d'avant-pied (A) comprend une première courbe à section transversale (D) à l'arrière de la plaque incurvée, une deuxième courbe à section transversale (E) au milieu de la plaque incurvée, et une troisième courbe à section transversale (F) à l'avant de la plaque incurvée ; et la plaque incurvée en fibre de carbone d'avant-pied (A) comprend une portion d'arc arrière (4c), une portion médiane de surface incurvée (4d), une première portion avant et une deuxième portion avant qui sont raccordées de manière séquentielle, dans lequel la première courbe en section transversale (D) est disposée sur une surface de raccordement entre la portion d'arc arrière (4c) et la portion médiane de surface incurvée (4d), la deuxième courbe à section transversale (E) est disposée sur une surface de raccordement entre la portion médiane de surface incurvée (4d) et la première portion avant, la troisième courbe à section transversale (F) est disposée sur une surface de raccordement entre la première portion avant et la deuxième portion avant, et un avant-orteil gauche (4e) et un avant-orteil droit (4f) sont disposés sur la première portion avant et la deuxième portion avant.

9. Pied prothétique selon la revendication 8, dans lequel la première courbe à section transversale (D) est une courbe en arc, et a un rayon d'arc $r_1$ de 30-35 mm et un angle central $\theta_1$ de 50°-60° ;

une expression mathématique de la deuxième courbe à section transversale (E) est :

$$y = -0{,}00009x^3 + 0{,}015x^2 + 0{,}02x\ 31{,}5\ ;$$

et

une expression mathématique de la troisième courbe à section transversale (F) est :

$$y = -0,000055x^3 + 0,006x^2 + 0,05x + 14,5.$$

**10.** Pied prothétique selon la revendication 9, dans lequel les formes de la deuxième courbe en section transversale (E) et de la troisième courbe en section transversale (F) sont dessinées selon une forme de base d'un arc transversal du corps humain, et ont une plus grande courbure des côtés intérieurs et une plus petite courbure des côtés extérieurs.

**11.** Pied prothétique selon la revendication 9, dans lequel la portion d'arc arrière (4c) est une surface cylindrique en arc qui est commandée par la première courbe à section transversale (D) et a une épaisseur de 4-5 mm ; le premier trou gauche (4a) et le premier trou droit (4b) sont prévus dans une partie arrière de la portion d'arc arrière (4c).

**12.** Pied prothétique selon la revendication 9, dans lequel la portion médiane de surface incurvée (4d) est une surface incurvée formée par la première courbe à section transversale (D) et la deuxième courbe à section transversale (E).

**13.** Pied prothétique selon la revendication 9, dans lequel une partie avant entière de la plaque incurvée en fibre de carbone d'avant-pied (A) est une surface incurvée formée par la deuxième courbe à section transversale (E) et la troisième courbe à section transversale (F), et une partie la plus basse de celle-ci est une surface en arc rectiligne vers le haut et est divisée en l'avant-orteil gauche (4e) et l'avant-orteil droit (4f) par une mince rainure (41) au milieu d'une partie avant de la plaque incurvée en fibre de carbone d'avant-pied (A) ; une épaisseur de la portion médiane de surface incurvée (4d) aux extrémités supérieures de l'avant-orteil gauche (4e) et de l'avant-orteil droit (4f) varie uniformément, de grande à petite, de l'arrière vers l'avant, l'épaisseur maximale est identique à l'épaisseur de la portion d'arc arrière (4c), et une épaisseur minimale est de 2,5 mm.

**14.** Pied prothétique selon la revendication 9, dans lequel la courbe longitudinale (G) d'avant-pied comprend une première section de courbe longitudinale (4g) d'avant-pied et une deuxième section de courbe longitudinale (4h) d'avant-pied, la première section de courbe longitudinale (4g) d'avant-pied est une section de ligne rectiligne avec un angle d'inclinaison de 23°-30°, et la deuxième section de courbe longitudinale (4h) d'avant-pied est un arc, ayant un rayon d'arc $r_2$ de 70-80 mm et un angle central $\theta_2$ de 50°-60°, et étant situé à une extrémité de queue de la partie avant de la courbe longitudinale (G) d'avant-pied, et la première section de courbe longitudinale (4g) d'avant-pied et la deuxième section de courbe longitudinale (4h) d'avant-pied sont en raccordement tangent fluide.

**Fig. 1**

**Fig. 2**

Fig. 3

Fig. 4

**Fig. 5**

**Fig. 6**

**Fig. 7**

**Fig. 8**

**Fig. 9**

Front

7a

7b

7c

7d

7g 7f 7e

**Fig. 10**

Front

8a

8b

**Fig. 11**

6

7

A

8

B

G

Front

H

**Fig. 12**

**EP 3 981 366 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150289996 A1